Europäisches Patentamt

(19) European Patent Office

Office européen des brevets

(11) Publication number: **0 305 170**
**A2**

# EUROPEAN PATENT APPLICATION

(21) Application number: 88307850.3

(22) Date of filing: 24.08.88

(51) Int. Cl.⁴: **A 61 F 9/00**
A 61 B 1/06

(30) Priority: 24.08.87 US 88879

(43) Date of publication of application:
01.03.89 Bulletin 89/09

(84) Designated Contracting States: DE FR GB

(71) Applicant: ALLERGAN INC
2525 Dupont Drive
Irvine California 92715 (US)

(72) Inventor: Willis, Timothy R.
2525 Dupont Drive
Irvine, CA 92715 (US)

(74) Representative: Day, Jeremy John et al
REDDIE & GROSE 16 Theobalds Road
London, WC1X 8PL (GB)

(54) Ophthalmic viewing instrument.

㉕ A surgical instrument for use in viewing a selected interior region of the eye includes a probe extending along a longitudinal probe axis from a proximal end portion to a distal end portion, the distal end portion of which probe is adapted to be inserted through an opening in an eye to a selected interior region of the eye. A first optical fiber extends within the probe to the distal end portion for use in propagating light obtained from a separate light source through the probe to the selected interior region of the eye for illuminating purposes. A second optical fiber extends within the probe to the distal end portion for use in transmitting an image from the selected interior region of the eye through the probe toward separate viewing instrumentation for visualization purposes. The instrument may also include a handpiece to facilitate probe handling and placement, and a laser delivery fiber to enable lens excision. The probe or the second optical fiber may have an offset tip to facilitate viewing in a direction offset from the probe axis.

FIG 4

EP 0 305 170 A2

# Description

## Ophthalmic Viewing Instrument

### Background of the Invention

#### Technical Field

This invention relates generally to surgical instruments, and more particularly to a new and improved viewing instrument for ophthalmic surgery.

#### Background Information

Ophthalmic surgery involves very precise, delicate surgery within the eye that presents unique viewing problems to the surgeon. The placement of an intraocular lens, for example, often requires proper positioning within the eye of haptics or other fine fixation members extending from the lens, and the surgeon may desire to view the eye interior both as placement proceeds and to verify that proper positioning has been accomplished.

Viewing is equally important for other ophthalmic procedures, particularly behind the iris, and it is complicated by the small incisions used to access the eye interior. Thus, the instruments utilized in this regard are of recognized significance, and each detail of their construction and operation of corresponding importance.

Both U.S. Patent No. 4,551,129 to Coleman et al. and U.S. Patent No 4,222,375 to Martinez describe devices for illuminating and irrigating during ophthalmic operations. However, these devices do not include viewing capabilities. They just illuminate. In addition, they may be somewhat cumbersome to use and not entirely effective for use in viewing the eye interior where desired.

Consequently, it is desirable to have a new and improved ophthalmic viewing instrument for purposes of viewing the eye interior. In particular, it is desirable to have such an instrument that is suitable for verifying proper placement of intraocular lens fixation members. In addition, a suitable instrument would enable viewing problems such as UGH syndrome or dislocation of an intraocular lens.

### Summary of the Invention

This invention recognizes the problems associated with the prior art and provides a new and improved ophthalmic viewing instrument with the desired attributes. Briefly, the above and further objects of the present invention are realized by providing a pen-size instrument that utilizes fiber optic technology to both illuminate and view selected portions of the eye interior. Thus, the invention achieves the desired functionality in a single, more conveniently manipulated instrument that is particularly adapted for use in ophthalmic surgery.

Generally, the instrument includes a probe that extends along a longitudinal probe axis from a proximal end portion of the probe to a distal end portion of the probe. The distal end portion of the probe is adapted to be inserted through an opening in an eye to a selected interior region of the eye. It may be inserted through a puncture hole at the periphery of the cornea, for example.

A first or illuminating optical fiber, extends within the probe to the distal end portion. It serves the function of propagating light obtained from a separate source to the distal end portion of the probe for purposes of illuminating selected portions of the eye interior. In addition, a second or viewing optical fiber extends within the probe to the distal end portion, and it is used for propagating light from the selected interior region of the eye through the probe toward separate viewing instrumentation for visualization purposes.

According to one aspect of the invention, there is also provided a laser delivery fiber disposed within the probe for use in subjecting tissue to laser energy. This enables such operative procedures as laser excision of the natural lens under illuminated viewing with one handheld instrument.

According to another aspect of the invention, the viewing fiber optic has an offset tip portion terminating in a distal end that faces radially outward from the distal end portion of the probe relative to the probe axis. A plurality of these viewing fibers may be employed, and they facilitate viewing of a region within the eye disposed in a position offset from the probe axis.

According to yet another aspect, there is provided an offset tip portion of the probe for this purpose. The offset tip portion extends along an axis that is angularly offset from the probe axis. It facilitates probe placement so that both the distal end of the illuminating fiber optic and the distal end of the viewing fiber optic face otherwise hard-to-access regions of the eye interior.

A method of viewing a selected interior region of an eye according to the invention includes making an incision in the eye through which to insert a probe within which is disposed a first optical fiber for illuminating purposes and a second optical fiber for viewing purposes. The method proceeds by inserting a distal end portion of the probe through the incision into the eye so that the distal end portion is in proximity with the selected region.

Then, light is propagated through the first optical fiber to illuminate the selected interior region of the eye, and an image from the selected interior region of the eye is propagated through the second optical fiber from the selected interior region of the eye toward separate viewing instrumentation for visualization purposes.

If the probe has the offset tip, the method may include the step of rotating the probe about the probe axis so that the second optical fiber faces the selected interior region of the eye. This rotation, which may be through any desired arc, enables the selected interior region to be scanned or for the desired portion thereof to be viewed.

The probe may include a laser delivery fiber for purposes of delivering laser energy for lens excision or other therapeutic laser delivery purposes. In that case, the method may include the step of propagating laser energy through the laser delivery fiber to subject the lens or other tissue to the laser energy.

The above mentioned and other objects and features of this invention and the manner of attaining them will become apparent, and the invention itself will be best understood, by reference to the following description taken in conjunction with the accompanying illustrative drawings.

Brief Description of the Drawings

FIGURE 1 of the drawings is a diagrammatic view of an ophthalmic viewing instrument constructed according to the invention that is in position for viewing a selected region of the eye interior;

FIGURE 2 is a perspective view of the distal end portion of the viewing instrument, with a portion broken away to expose details of the interior;

FIGURE 3 is a perspective view of the distal end portion of a second embodiment that includes a laser delivery fiber;

FIGURE 4 is a perspective view of the distal end portion of a third embodiment in which the viewing fiber optics include offset tip portions; and

FIGURE 5 is a perspective view of the offset probe tip of a fourth embodiment.

Description of the Preferred Embodiments

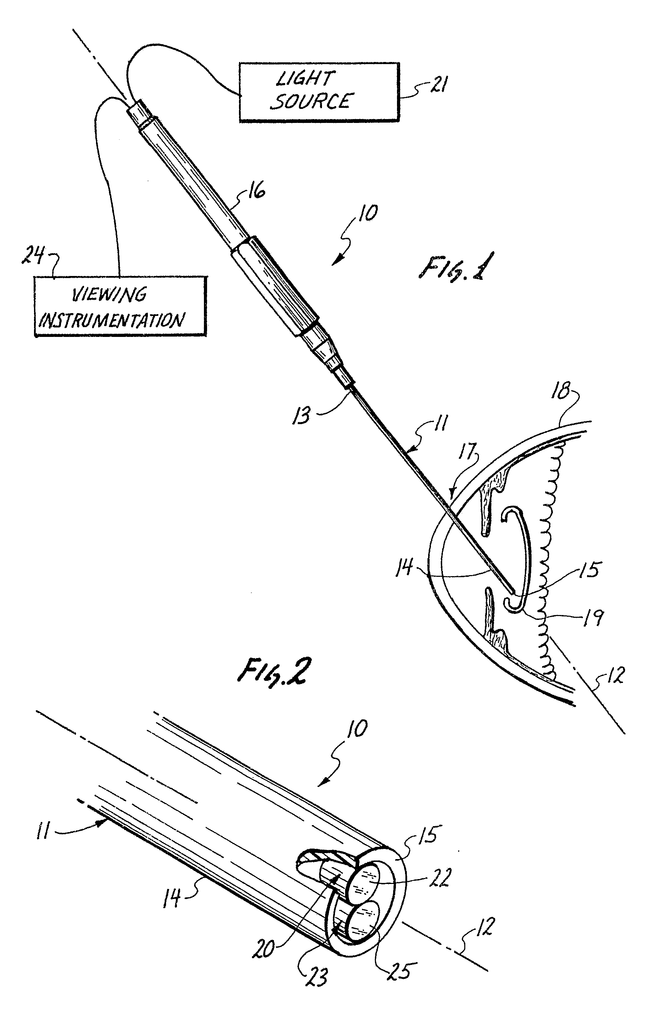

Referring now to Figs. 1 and 2, there is shown a new and improved ophthalmic viewing instrument 10 constructed according to the invention. Generally, the instrument 10 includes a probe 11 extending along a probe axis 12 from a proximal end portion 13 of the probe 11 to a distal end portion 14 of the probe 11 (Fig. 1). The distal end portion 14 extends to a distal end 15 of the probe 11, and the proximal end portion 13 is attached to a handpiece 16 that is adapted to be held in the hand of a user. It is attached by suitable known means, such as a coupling that enables probe replacement.

The distal end portion 14 of the probe 11 is adapted to be inserted through an opening in an eye to a selected interior region of the eye, i.e. it has a suitable size and shape and may include a pointed tip that facilitates entry by puncturing. It is illustrated inserted through an opening 17 in the form of a puncture hole at the periphery of the cornea of a human eye 18, and it extends through the opening to a position in proximity with a selected interior region of the eye 18. In Fig. 1, the selected interior region is adjacent the capsular bag 19 of the eye 18.

As an idea of the structure and size employed, the handpiece 16 may be a polished and hard anodized aluminum material that is approximately ten centimeters long and six to seven centimeters in diameter. The probe 11 may be a furnace brazed stainless steel cannula-like tube about ten to twenty millimeters long and one-half to three millimeters in diameter. Fabrication is accomplished according to known techniques to provide a size and shape that is conveniently held in the hand and manipulated by the user during ophthalmic surgery. Of course, the above dimensions may vary within the inventive concepts disclosed according to the precise application.

Disposed within the probe 11 is a first or illuminating optical fiber 20 (Fig. 2). It extends within the probe 11 from the proximal end 13 to the distal end portion 14, and to the distal end 15 of the probe 11 in the illustrated instrument 10. The illuminating optical fiber 20 is fabricated and disposed within the probe 11 according to known techniques and functions as illuminating means for use in propagating light obtained from a separate light source 21 (Fig. 1) through the probe 11 and out of a distal end 22 of the illuminating optical fiber 20 to the selected interior region of the eye for illuminating purposes.

In the illustrated instrument 10, the distal end 22 of the illuminating optical fiber 20 and the distal end 15 of the probe 11 face a common direction, i.e. a direction toward which the probe axis 12 extends. Other alignments may be employed. Regarding the light source 21, it represents any of various known sources suitable for propagating light through the illuminating optical fiber 20 for illuminating purposes.

A second or viewing optical fiber 23 is also disposed within the probe 11 (Fig. 2). The optical fiber 23 is an imaging fiber and it extends within probe 11 to the distal end portion 14, and to the distal end 15 of the probe 11 in the illustrated instrument 10. The viewing optical fiber 23 is also fabricated and disposed within the probe 11 according to known techniques, and functions as viewing means for use in transmitting an image through the probe 11 (reflected light received through a distal end 25 from the selected interior region) toward separate viewing instrumentation 24 (Fig. 1) for visualization purposes.

Like the distal end 22 of the illuminating optical fiber 20, the distal end 25 of the viewing optical fiber 23 faces a common direction with the distal end 15 of the probe 11. However, other alignments may be employed as subsequently described. Regarding the viewing instrumentation 24, it represents any of various types of instrumentation, such as an eye piece, microscope, camera, video monitor, slit lamp, or computer assisted visualization equipment, and the like.

Operationally, in order to view a selected interior region of an eye, the distal end portion 14 of the probe 11 is inserted through an opening into the eye so that the distal end portion 14 is in proximity with a selected region of the eye interior. Then light is propagated from the separate light source 21 through the illuminating optical fiber 20 to illuminate the selected interior region of the eye, and an image of the scene adjacent the distal end 25 of the viewing optical fiber 25 light is propagated through the viewing optical fiber 23 from the selected interior region of the eye toward the viewing instrumentation 24 for visualization purposes.

Considering now Fig. 3, there is shown a second embodiment of the invention, an instrument 100. For convenience, many of the reference numerals have been increased by one hundred over those designating similar elements in Figs. 1 and 2. These are not described in further detail.

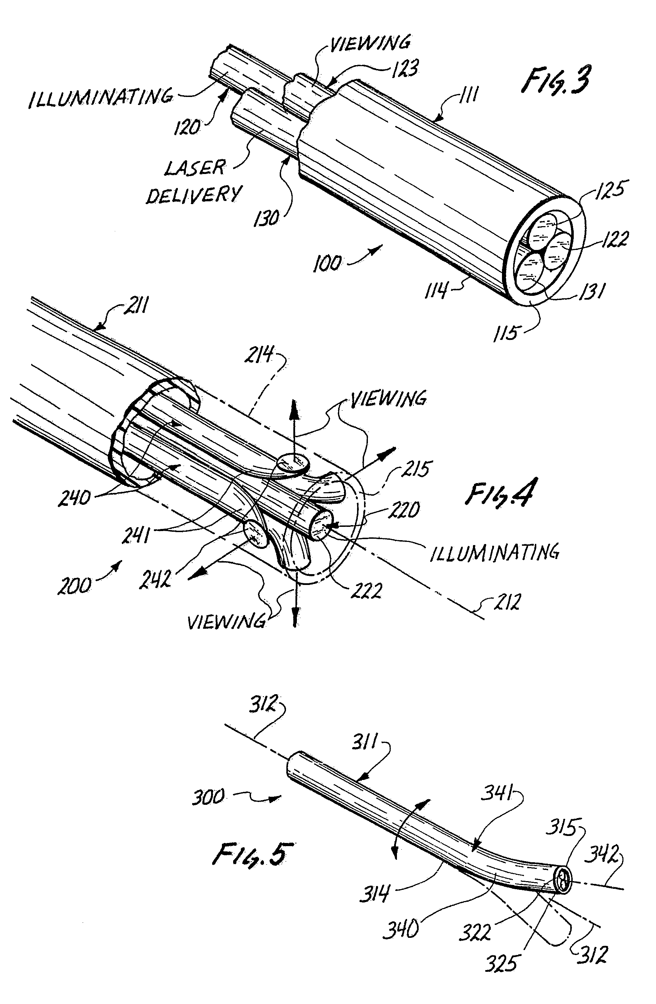

In addition to the similar elements, the instrument 100 includes a laser delivery fiber 130 disposed within the probe 111. It extends within the probe 111 to a distal end portion 114, and to a distal end 115 of the probe 111 in the illustrated instrument 100. The laser delivery fiber 130 is fabricated and disposed within the probe 111 according to known techniques. It functions as laser delivery means for use in propagating laser energy obtained from a separate laser source (not shown) through the probe 111 to the selected interior region of the eye for purposes of subjecting tissue within the eye to laser energy. The laser delivery fiber may also be used in the embodiments of Figs. 4 and 5, if desired.

Operationally, the distal end portion 114 of the probe 111 is inserted into the eye in the manner described above for the instrument 10. This is done so that the distal end portion 114 is in proximity with tissue to be subjected to laser energy. For excision of the natural lens, the distal end portion 114 is advanced to a position in proximity with the lens.

With the distal end portion 114 so positioned, light is propagated through the first or illuminating fiber optic 120 to illuminate the tissue, an image is propagated through the second or viewing optical fiber 123 from the lens or other tissue toward separate viewing instrumentation for visualization purposes, and laser energy is propagated through the laser delivery or third optical fiber 130, distally of the distal end 131 to subject the lens or other tissue lens to laser energy.

A third embodiment, instrument 200, is illustrated in Fig. 4. For convenience, many of the reference numerals have been increased by two hundred over those designating similar elements in Figs. 1 and 2. These are not described in further detail.

In addition to the similar elements, the instrument 200 includes a plurality of one or more second or viewing optical fiber 240 that are generally similar to the second or viewing fiber optic 23 of the instrument 10. Unlike the viewing optical fiber 23, each of the viewing optical fibers 240 includes a radially facing tip portion 241 extending through a port in the probe 211 to a distal end 242 that faces generally radially outward relative to a probe axis 212 as depicted by the radially outward extending arrows in Fig. 4. So configured, each one of the tip portions 241 functions as radial viewing means for facilitating viewing within the eye interior radially outward from the distal end portion 214 of the probe 211 relative to the probe axis 212.

Operationally, the probe 211 is inserted into the eye and light is propagated through the illuminating optical fiber 220. Although directed in a common direction with the direction of the probe axis 212, the light is dispersed within the eye so that a sufficient amount is reflected for viewing purposes from tissue faced by the viewing optical fibers 240. Images are transmitted through the viewing optical fibers 240 to viewing instrumentation (not shown). In addition, the probe 211 may be rotated slightly so that one of the distal ends 242 of the viewing optical fibers 240 faces a region to be viewed. Thus, the instrument 200 facilitates viewing radially relative to the probe axis 212.

A fourth embodiment, instrument 300, is illustrated in Fig. 5 that also facilitates radial viewing. For convenience, many of the reference numerals have been increased by three hundred over those designating similar elements in Figs. 1 and 2. These are not described in further detail.

Unlike the probe 11 of the instrument 10, the probe 311 of the instrument 300 includes an offset tip portion 340 of a distal end portion 314 of the probe 311. The offset tip portion 340 extends from a setback region 341 of the distal end portion 314 to the distal end 315 of the probe 311 along an offset axis 342 that is angularly offset from the probe axis 312. So configured, the offset tip portion 340 functions as offsetting means for facilitating advantageous placement of the distal end portion 314 of the probe 311 within the eye interior. It can be placed in a position such that a common direction faced by the distal end 315 of the probe 311, a distal end 321 of an illuminating optical fiber, and a distal end 325 of a viewing optical fiber, are out of alignment with the probe axis 312.

Operationally, the distal end portion 314 of the probe 311 is inserted into the eye. Then, the probe 311 is rotated as indicated by the double-headed arrow in Fig. 5, so that the distal end 315 faces along the offset axis 342 toward a selected region of the eye interior that is out of line with the probe axis 312.

Thus, the invention provides a pen-size instrument that utilizes fiber optic technology to both illuminate and view selected portions of the eye interior. It thereby overcomes problems associated with the prior art in a single, more conveniently manipulated instrument that is particularly adapted for use in ophthalmic surgery.

Although an exemplary embodiment of the invention has been shown and described, many changes, modifications, and substitutions may be made by one having ordinary skill in the art without necessarily departing from the spirit and scope of this invention.

**Claims**

1. An ophthalmic instrument, comprising:
a probe extending along a longitudinal probe axis from a proximal end portion of the probe to a distal end portion of the probe, the distal end portion of which probe is dimensioned and arranged to be inserted through an opening in an eye to a selected interior region of the eye;
illuminating means, including a first optical fiber extending within the probe to the distal end portion, for use in propagating light obtained from a separate light source through the probe to the selected interior region of the eye for illuminating purposes; and
viewing means, including a second optical fiber extending within the probe to the distal end portion, for use in propagating an image from the selected interior region of the eye through the probe toward separate viewing instrumentation for visualization purposes;

the second optical fiber having a tip portion that faces along an axis that is angularly offset from the probe axis.

2. An instrument as recited in Claim 1, wherein the tip portion of the second optical fiber faces generally radially away from the probe.

3. An instrument as recited in Claims 1 or 2, wherein the viewing means includes:

a plurality of second optical fibers extending within the probe to the distal end portion of the probe, each one of which second optical fibers includes a radially facing tip portion to facilitate viewing radially outward from the distal end portion of the probe in a plurality of directions.

4. An instrument as recited in Claim 3, wherein: the radially facing tip portions of the plurality of second optical fibers face radially in generally equally spaced-apart directions.

5. An instrument as recited in Claims 3 or 4, wherein:

the plurality of second optical fibers includes four optical fibers; and

the radially facing tip portions of the four optical fibers face in directions spaced apart approximately ninety degrees.

6. An instrument as recited in Claim 1, wherein: the second optical fiber extends to a distal end of the probe, the second optical fiber and the distal end generally face a common direction, and the probe has an offset tip portion such that the common direction is angularly offset from the probe axis.

7. A method of viewing a selected interior region of an eye, comprising:

inserting a distal end portion of a probe through an opening into an eye so that the distal end portion is in proximity with the selected region, within which probe is disposed a first optical fiber for illuminating purposes and a second optical fiber for viewing purposes;

propagating light through the first optical fiber to illuminate the selected interior region of the eye; and propagating an image of at least a portion of the selected region through the second optical fiber from the selected region of the eye toward separate viewing instrumentation for visualization purposes;

wherein the method further includes;

providing an offset tip on the probe such that the second optical fiber faces a direction out of alignment with a probe axis of the probe; and rotating the probe about the probe axis so that the second optical fiber faces the selected region of the eye.

8. An ophthalmic instrument, comprising: a handpiece extending between proximal and distal end portions of the handpiece;

a rigid probe extending along a longitudinal probe axis from a proximal end portion of the probe to a distal end portion of the probe;

the proximal end portion of the probe being attached to the distal end portion of the handpiece and the distal end portion of the probe being dimensioned and arranged to be inserted through an opening in an eye to a selected interior region of the eye;

the handpiece being adapted to be held in the hand of a user to facilitate probe handling and the placement of the distal end portion of the probe within the eye interior;

illuminating means, including a first optical fiber extending within the probe to the distal end portion of the probe, for use in propagating light obtained from a separate light source through the probe to the selected interior region of the eye for illuminating purposes; and

viewing means, including a second optical fiber extending within the probe to the distal end portion of the probe, for use in propagating an image from the selected interior region of the eye through the probe toward separate viewing instrumentation for visualization purposes; and

the first and second fibers extending generally axially out of the proximal end of the handpiece.

9. An ophthalmic instrument as recited in Claim 8, further comprising:

laser delivery means, including a laser delivery fiber. extending within the probe to the distal end portion of the probe, for use in propagating laser energy obtained from a separate laser source through the probe to the selected interior region of the eye for purposes of subjecting tissue within the eye to laser energy.

10. An ophthalmic instrument as recited in Claim 8, wherein the viewing optical fiber has:

a radially facing tip portion for facilitating viewing within the eye interior generally radially outward from the distal end portion of the probe relative to the probe axis.

LIGHT
SOURCE
21

24

VIEWING
INSTRUMENTATION

16

10

FIG.1

13

11

18

17

14

15

19

12

FIG.2

10

11

15

22

14

20

23

25

12

VIEWING

*FIG.3*

ILLUMINATING

123

111

120

LASER
DELIVERY

130

100

125

122

131

114

115

211

214

VIEWING

240

215

220

241

222

200

242

ILLUMINATING

212

VIEWING

*FIG.4*

312

311

300

341

315

342

314

340

322

312

*FIG.5*

325